# EUROPEAN PATENT SPECIFICATION

(11) **EP 1 173 549 B1**
(45) Date of publication and mention of the grant of the patent: **13.08.2008**
(21) Application number: 00941122.4
(22) Date of filing: 05.05.2000
(51) Int. Cl.: C12N 5/06, C12N 5/08, A61K 35/14, A61P 37/00

(54) **USE OF CYTOKINES AND MITOGENS TO INHIBIT PATHOLOGICAL IMMUNE RESPONSES**
VERWENDUNG VON ZYTOKINEN UND MITOGENEN ZUR INHIBIERUNG VON PATHOLOGISCHEN IMMUNANTWORTEN
UTILISATION DE CYTOKINES ET DE MITOGENES POUR EMPECHER DES REPONSES IMMUNITAIRES PATHOLOGIQUES

(30) Priority: 05.05.1999 US 132616 P
(43) Date of publication of application: 23.01.2002
(73) Proprietor: UNIVERSITY OF SOUTHERN CALIFORNIA, Los Angeles, CA 90007-4344 (US)
(72) Inventor: HORWITZ, David, A., Santa Monica, CA 90402 (US)
(74) Representative: Kiddle, Simon John
(86) International application number: PCT/US2000/012284
(87) International publication number: WO 2000/066158

(56) References cited:
- WO-A-99/25366
- H.-Y. HAN ET AL.: "A new type of CD4+ suppressor T cell completely prevents spontaneous autoimmune diabetes and recurrent diabetes in syngeneic islet-transplanted NOD mice." JOURNAL OF AUTOIMMUNITY, vol. 9, 1996, pages 331-339, XP002153791 new york, n.y., us
- J.D. GRAY ET AL.: "The role of transforming growth factor beta in the generation of suppression: An interaction between CD8+ T and NK cells." JOURNAL OF EXPERIMENTAL MEDICINE, vol. 180, no. 5, 1 November 1994 (1994-11-01), pages 1937-1942, XP000961145 new york, n.y., us
- J.D. GRAY ET AL.: "Generation of an inhibitory circuit involving CD8+ T cells, IL-2, and NK cell-derived TGF-beta: contrasting effects of anti-CD2 and anti-CD3" JOURNAL OF IMMUNOLOGY,US,THE WILLIAMS AND WILKINS CO. BALTIMORE, vol. 160, no. 5, March 1998 (1998-03), pages 2248-2254, XP002099413 ISSN: 0022-1767 cited in the application
- A.H. ROOK ET AL.: "Effects of transforming growth factor beta on the functions of natural killer cells: Depressed cytolytic activity and blunting of interferon responsiveness." THE JOURNAL OF IMMUNOLOGY, vol. 136, no. 10, 15 May 1986 (1986-05-15), pages 3916-3920, XP002153792 BALTIMORE, US

## Description

### FIELD OF THE INVENTION

The field of the invention is generally related to the use of medicaments for treating cell-mediated autoimmune disorders.

### BACKGROUND OF THE INVENTION

Autoimmune diseases are caused by the failure of the immune system to distinguish self from non-self. In these diseases, the immune system reacts against self tissues and this response ultimately causes inflammation and tissue injury. Autoimmune diseases can be classified into two basic categories: antibody-mediated diseases such as systemic lupus erythematosus (SLE), pemphigus vulgaris, myasthenia gravis, hemolytic anemia, thrombocytopenia purpura, Grave's disease, Sjogren's disease and dermatomyositis; and cell-mediated diseases such as Hashimoto's disease, polymyositis, disease inflammatory bowel disease, multiple sclerosis, diabetes mellitus, rheumatoid arthritis, and scleroderma.

In many autoimmune diseases, tissue injury is caused by the production of antibodies to native tissue. These antibodies are called autoantibodies, in that they are produced by a mammal and have binding sites to the mammal's own tissue. Some of these disorders have characteristic waxing and waning of the amount of circulating autoantibodies causing varying symptoms over time.

Of the different types of antibody-mediated autoimmune disorders, SLE is a disorder that has been well studied and documented. SLE is a disorder of generalized autoimmunity characterized by 8 cell hyperactivity with numerous autoantibodies against nuclear, cytoplasmic and cell surface antigens. This autoimmune disease has a multifactorial pathogenesis with genetic and environmental precipitating factors (reviewed in Hahn, B.H., Dubois' Lupus Erythematosus, 5th Ed. (1997), pp. 69-76 (D.J. Wallace et al. eds., Williams and Wilkins, Baltimore)). Among the numerous lymphocyte defects described in SLE is a failure of regulatory T cells to inhibit B cell function (Horwitz, D.A., Dubois' Lupus Erythematosus, 5th Ed. (1997), pp. 155-194 (D.J. Wallace et al. eds., Williams and Wilkins, Baltimore)). Sustained production of polyclonal IgG and autoantibodies in vitro requires T cell help (Shivakumar, S. et al. (1989), J Immunol 143:103-112).

Regulatory T cells can down-regulate antibody synthesis by lytic or cytokine-mediated mechanisms. The latter involve transforming growth factor-beta (TGF-β) and other inhibitory cytokines (Wahl, S.M. (1994), J Exp Med 180:1587-190). Circulating B lymphocytes spontaneously secreting antibodies are increased in patients with active SLE (Klinman, D.M. et al. (1991), Arthritis Rheum 34:1404-1410).

Clinical manifestations of SLE include a rash (especially on the face in a "butterfly" distribution), glomerulonephritis, pleurisy, pericarditis and central nervous system involvement. Most patients are women, and are relatively young (average age at diagnosis is 29).

The treatment of SLE depends on the clinical manifestations. Some patients with mild clinical symptoms respond to simple measures such as nonsteroidal anti-inflammatory agents. However, more severe symptoms usually require steroids with potent anti-inflammatory and immunosuppressive action such as prednisone. Other strong immunosuppressive drugs which can be used are azathioprine and cyclophosphamide. The steroids and other immunosuppressive drugs have side effects due to the global reduction of the mammal's immune system. There is presently no ideal treatment for SLE and the disease cannot be cured.

Currently, considerable attention has been focused on the identity of genes which enhance the susceptibility or resistance to SLE, the identification of antigenic determinants that trigger the disease, the molecular mechanisms of T cell activation which results in survival or apoptosis, cytokines which determine T cell function, and the properties of the autoantibody-forming B cells. Many examples of T cell dysregulation in SLE have been described (reviewed in Horwitz, D.A. et al., Dubois' Lupus Erythematosus, 5th Ed. (1997), pp. 83-96 (D.J. Wallace et al. eds., Williams and Wilkins, Baltimore). Although it is well recognized that the primary role of certain lymphocytes is to down-regulate immune responses, progress in elucidating the identity and mechanisms required for generation of these cells has been slow.

Interleukin-2 (IL-2) has previously been considered to have an important role in the generation of antigen non-specific T suppressor cells. Anti-IL-2 antibodies given to mice coincident with the induction of graft-versus-host-disease resulted in several features of SLE (Via, C.S. et al. (1993), lnternational lmmunol. 5.565-572). Whether IL-2 directly or indirectly is important in the generation of suppression has been controversial (Fast, L.D. (1992), J. Immunol. 149:1510-1515; Hirohata, S. et al. (1989), J. Immunol. 142:3104-3112; Baylor, C.E. (1992), Advances Exp. Mod. Biol. 319:125-135). Recently, IL-2 has been shown to induce CD8⁺ cells to suppress HIV replication in CD₄+ T cells by a non-lytic mechanism. This effect is cytokine mediated, but the specific cytokine has not been identified (Kinter, A.L. et al. Proc. Natl. Acad. Sci. USA 92:10985-10989; Barker, T.D. et al. (1996), J. Immunol. 156:4478-4483). T cell production of IL-2 is decreased in SLE (Horwitz, D.A. et al. (1997), Dubois' Lupus Erythematosus, 5th Ed. (1997), pp. 83-96, D.J. Wallace et al. eds., Williams and Wilkins, Baltimore).

C_{D}8+ T cells from subjects with SLE sustain rather than suppress polyclonal IgG production (Linker-Israeli, M. et al. (1990), Arthritis Rheum. 33:1216-1225). CD8+ T cells from healthy donors can be stimulated to enhance antibody production (Takahashi, T. et al. (1991), Clin. Immunol. Immunopath. 58:352-365). However, neither IL-2 nor CD4+ T cells, by themselves, were found to induce CD8+ T cells to develop strong suppressive activity. When NK cells were included in the cultures, strong suppressive activity appeared (Gray, J.D. et al. (1994) J. Exp. Med. 180:1937-1942). It is believed that the contribution of NK cells in the culture was to produce transforming growth factor beta (TGF-β) in its active form. It was then discovered that non-immunosuppressive (2-10 pg/ml) concentrations of this cytokine served as a co-factor for the generation of strong suppressive effects on IgG and IgM production (Gray, J.D. et al. (1994) J. Exp. Med. 180:1937-1942). In addition, it is believed that NK cells are the principal source of TGF-β in unstimulated lymphocytes (Gray, J.D. et al. (1998), J. Immunol. 160:2248-2254).

TGF-βs are a multifunctional family of cytokines important in tissue repair, inflammation and immunoregulation (Massague, J. (1980), Ann. Rev. Cell Biol. 6:597). TGF-β is unlike most other cytokines in that the protein released is biologically inactive and unable to bind to specific receptors (Spom, M.B. et al. (1987) J. Cell Biol. 105:1039-1045). The latent complex is cleaved extracelluarly to release active cytokine as discussed below. The response to TGF-β requires the interaction of two surface receptors (TGF-β-R1) and TGF-β-R2) which are ubiquitously found on mononuclear cells (Massague, J. (1992), Cell 69:1067-1070 ). Thus, the conversion of latent to active TGF-β is the critical step which determines the biological effects of this cytokine.

It was found that SLE patients have decreased production of TGF-β1 by NK cells. Defects in constitutive TGF-β produced by NK cells, as well induced TGF-β were documented in a study of 38 SLE patients (Ohtsuka, K. et al. (1998), J. Immunol. 160:2539-2545). Neither addition of recombinant IL-2 or TNF-alpha, or antagonism of IL-10 normalized the TGF-β defect in SLE. Decreased production of TGF-β in SLE did not correlate with activity of disease and, therefore, may be a primary defect.

Han et al ((1996, J. Autoimmunity, 9:331-339) discloses that an auto reactive CD4+ T cell clone Isolated from diabetic NOD mice secretes TGFβ.

Systemic administration of TGF-β, IL-2. or a combination of both can lead to serious side effects. These cytokines have numerous effects on different body tissues and are not very safe to deliver to a patient systemically. It is, therefore, an object of the invention to provide methods and kits for treating mammalian cells that are responsible for controlling the regulation of autoantibodies to increase the population of cells that down regulate auto-antibody production.

Accordingly, the present invention provides use of peripheral blood mononuclear cells (PBMC) which have been obtained from a patient with a cell-mediated autoimmune disorder and treated with a regulatory composition comprising TGF-β, for the preparation of a medicament for treating said cell-mediated autoimmune disorder in said patient.

Thus, the present invention relates to treating cell-mediated autoimmune diseases. The medical use involves removing peripheral blood mononuclear cells (PBMC) from the patient and treating the cells with an regulatory composition for a time sufficient to suppress tissue injury by immune cells. The treated cells are then reintroduced to the patient, with a resulting amelioration of the autoimmune symptoms. The regulatory composition comprises TGF-β and agents which enable T cells to respond to TGF-β.

### BRIEF DESCRIPTION OF THE DRAWINGS

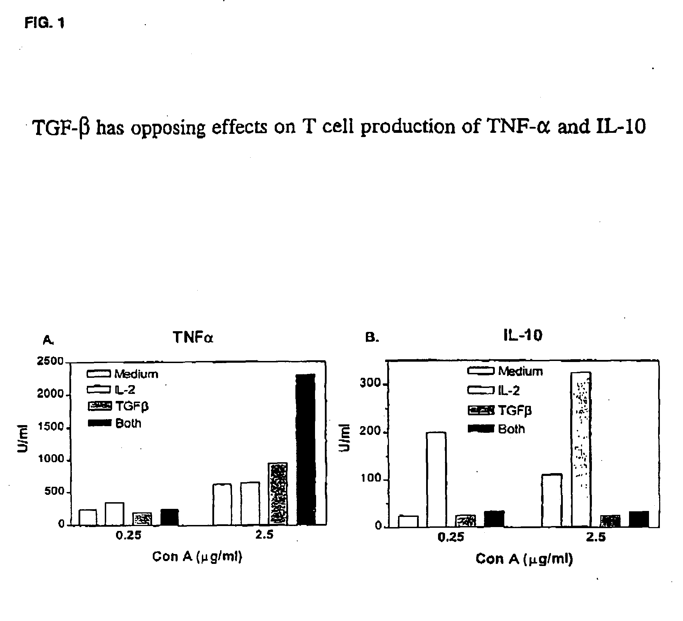

Figure 1 depicts the effects of TGF-β on T cell production of TNF-α and IL-10. Purified T cells ( 1 x 10⁵ cells/well) in serum free AIM V medium were added to flat bottomed microwells and stimulated with a low dose (0.5 µg/ml) or high dose (5 µg/ml) of Con A with or without IL-2 (10U/ml) in the presence or absence of TCF-β (1 ng/ml). Supernatants were collected at 2 days and 5 days and tested for TNF-α and IL-10 by ELISA. Maximal production of TGF-β was found at 2 days and for IL-10 at 5 days. TGF-β abolished IL-10 production and up-regulated TNF-α production.

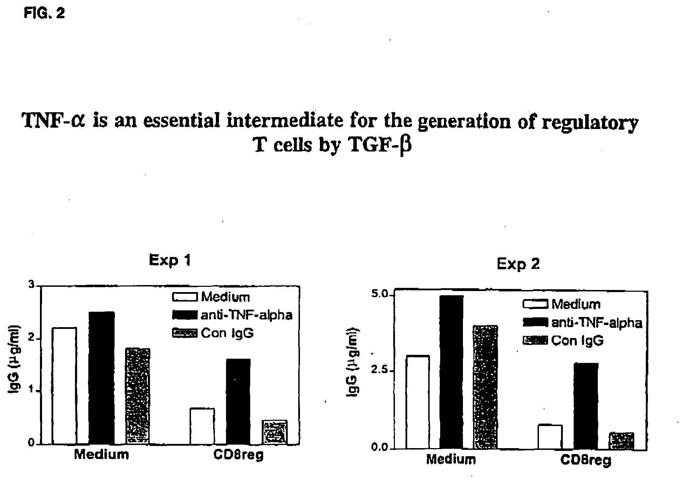

Figure 2 shows that TNF-α is an essential Intermediate for the generation of regulatory T cells by TGF-β. Purified CD8+ cells were incubated overnight with Con A (2.5 µg/ml). II-2 (10U) and TGF-β (10 pg/ml), After washing these cells were added to CD4+ B cells and stimulated with anti-CD2. To some wells anti-TNF-α antibody (10 µg/ml) or isotype control antibody (10 µg/ml) was included. After 7 days supernatants were evaluated for IgG content by an ELISA. The regulatory activity of conditioned CD8+ cells was reversed by anti-TNF-α.

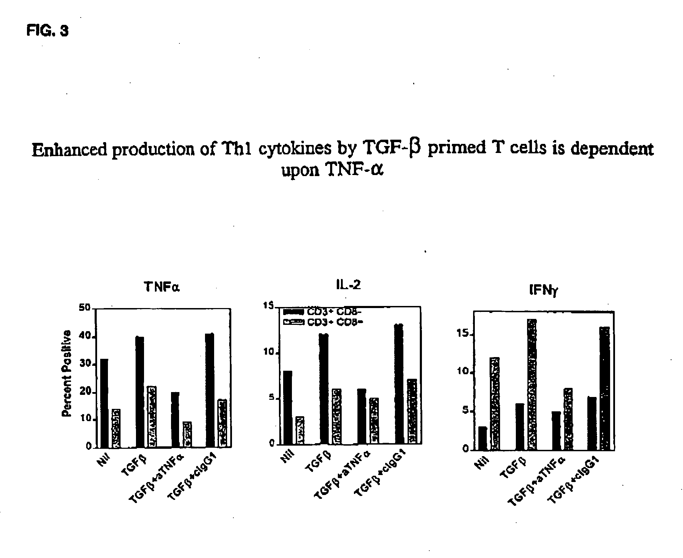

Figure 3 depicts that enhanced production of Th1 cytokines by TGF-β primed T cells is dependent upon TNF-α. Purifed naive T cells were cultured with Con A (5µg/ml) an IL-2 (10U/ml) in the presence of TGF-β (1 ng/ml). Some wells also received neutralizing anti-TNF-α antibody (10 µg/ml) or isotype control antibody (10 µg/ml). After 5 days of culture, the cells were washed and replated at 1 x 10⁵ cells/well in fresh medium. The next day they were restimulated with Con A and IL-2 for 6 hours and, in the presence of brefeldin A (10 µg/ml), the cells were stained for CD8 and the cytokines indicated. The percentage of CD8+ and CD8- cells expressing TNF-α, IL-2 and IFN-γ is shown. Note that neutralization of TNF-α in primary cultures abolished the enhancing effects of TGF-β on production of Th1 cytokines.

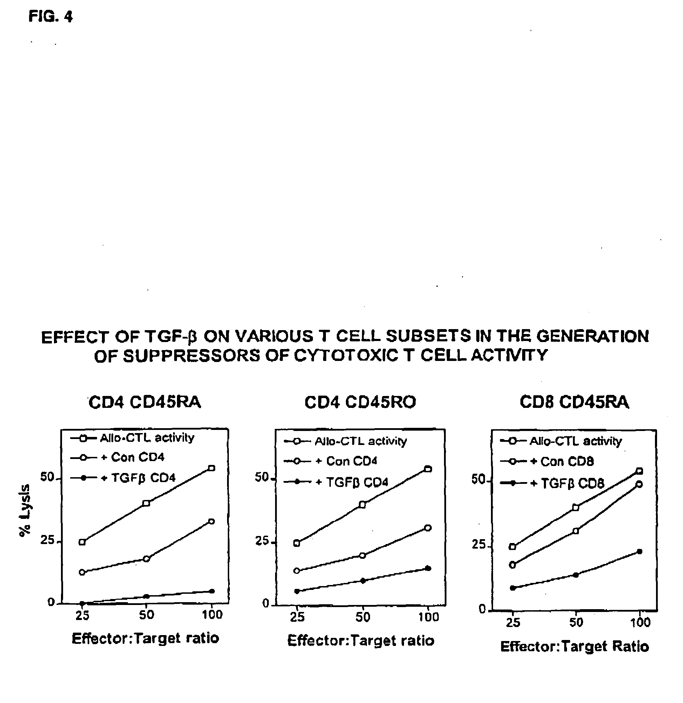

Figure 4 depicts the effect of TGF-β in generating suppressors of cytotoxic T cell activity. T cells from donor A prepared by E rosetting were divided into two portions. One portion was used as responders for an allogeneic mixed lymphocyte reaction (allo-MLR). The other portion was used to prepare the T cell subsets indicated by negative selection after staining the cells with appropriate monoclonal antibodies and removing the stained calls using immunomagnetic beads. The responder T cells were mixed with stimulator cells from donor B (irradiated T cell depleted peripheral blood mononuclear cells) and cultured for 5 days to generate killer cells. Contols consisted of the T cell subsets cultured for 5 days with or without stimulator cells. Afterwards, the cells were washed, counted and used to assess allo-cytotoxic T cell activity. The responder cells from donor A were mixed with chromium labeled lymphoblasts from donor B in the effector to target cell ratios shown and chromium release was measured in a standard 4 hour assay (open squares). T cells subsets cultured with stimulators were added in a ratio of 1 regulatory cell per 4 responder cells (open circles). T cell subsets cultured with stimulators with TGF-beta are shown as closed circles. In all experiments, the maximal effects of TGF-beta were on naive CD4 CD45RA+ CD45RO- cells.

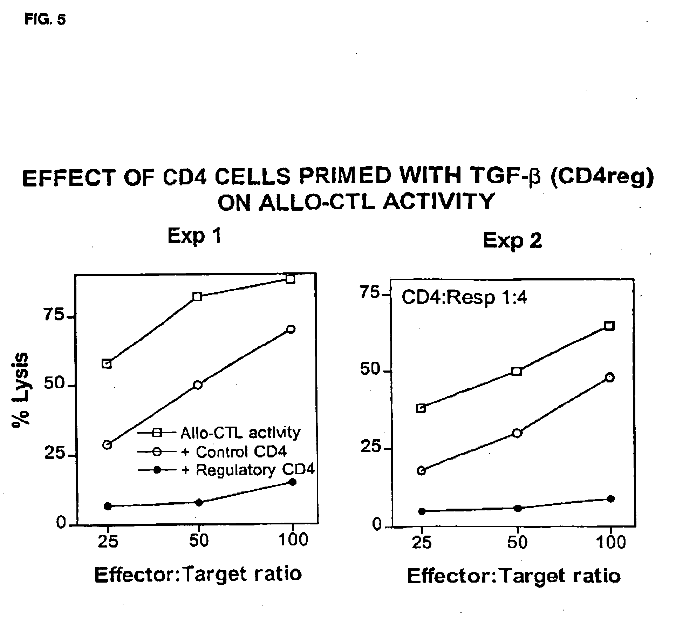

Figure 5 depicts the effect of CD4 cells primed with TGF-beta on allo-cytotoxic T lymphocyte (CTL) activity. The addition of CD4 CD45RA cells that had been cultured for 5 days without stimulators had no effect on CTL activity (result not shown). Culturing these T cells with stimulator cells resulted in modest to moderate suppressive activity. In all experiments, culture of these T cells with TGF-beta 1 ng/ml markedly suppressed, or abolished allo-CTL activity.

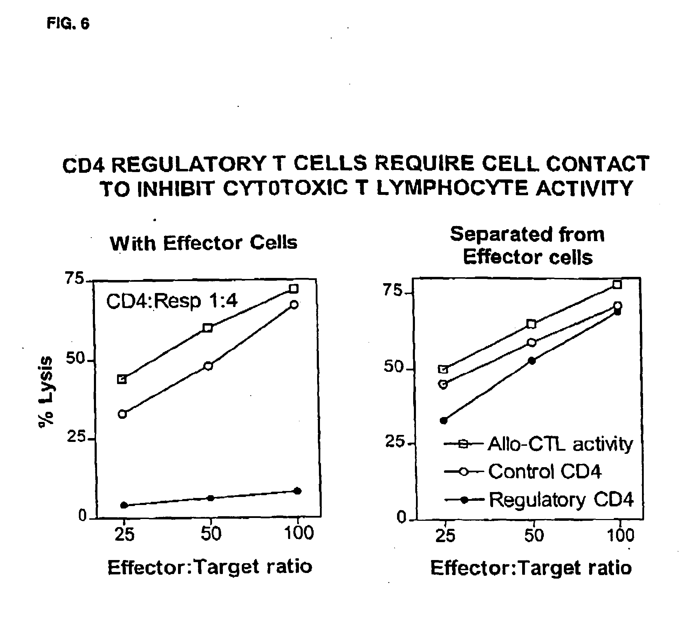

Figure 6 demonstrates that regulatory T cells require cell contact to inhibit CTL activity. Regulatory CD4 cells were prepared from CD4 CD45RA cells cultured with TGF-beta as described above. Some of these cells were mixed with responder and chromium-labeled target cells, while others were separated from the killer cells by a membrane. Inhibition of cytotoxic T lymphocyte activity (CTL) was only observed when the regulatory T cells were in direct contact with the killer cells.

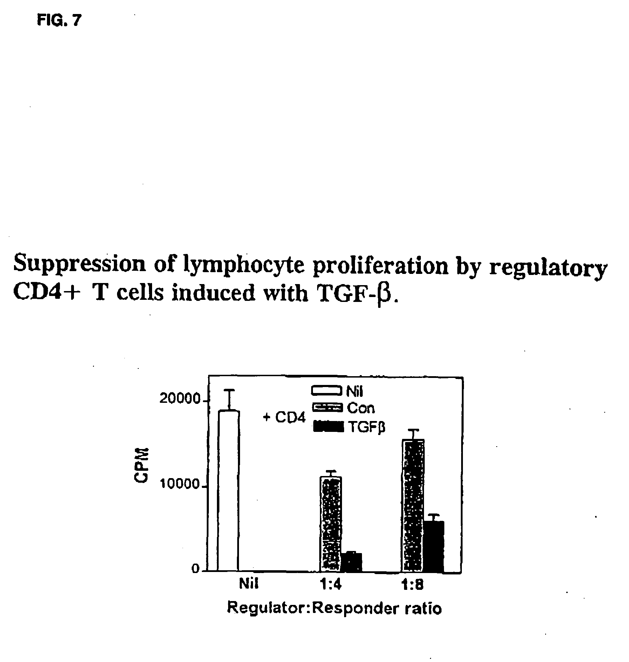

Figure 7 depicts suppression of lymphocyte proliferation by regulatory CD4+ T cells induced with TGF-β. Naive CD4+ T cells from donor A were mixed with stimulator cells as described above and added to fresh responder and stimulator cells at the indicated ratios. The bars show the uptake of tritiated thymidine ± SEM after 7 days of culture. The lightly shaded bar (Nil) indicates the proliferative response of the responder T cells without added CD4+ cells. The darkly shaded bar indicates the effect of control CD4+ cells which had been cultured with stimulator cells without TGF-β. The black bar indicates the effect of CD4+ cells that had been mixed with stimulator cells in the presence of TGF-β (1 ng/ml). The effect of these CD4+ calls on the proliferative response of fresh responder cells added to irradiated stimulator cells after 7 days of culture is shown. The bars indicate the mean uptake of tritiated thymidine.

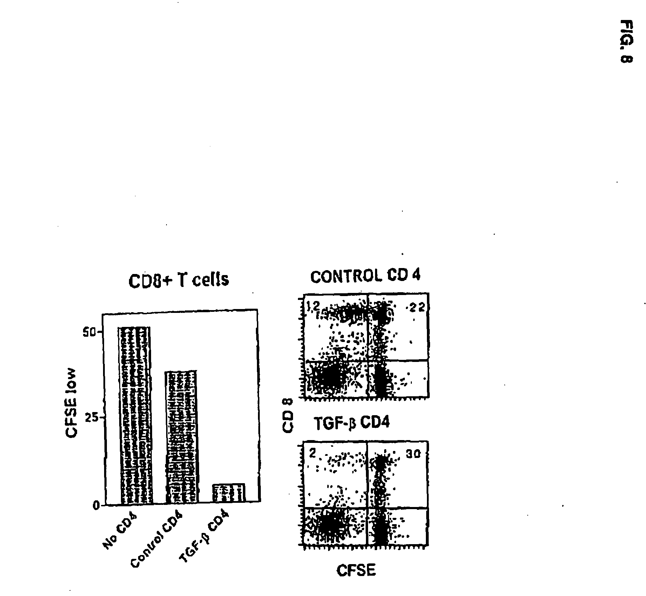

Figure 8 depicts the regulatory activity of CD25+ CD4 T cells. CD4+ cells were stimulated with irradiated allogeneic non-T cells ± TGF-β (1 ng/ml) for 5 days. After washing, the CD4+ cells were stained with DII and fresh responder T cells were stained with carboxyfluorescein (CFSE). control or TGF-β primed CD4+ cells were added to the responder T cells and allo-stimulator cells in a 1:4 ratio. After 5 days, the cells were harvested and analyzed by flow cytometry. The intensity of CFSE in CD8+ cells was determined by gating on DII negative cells. Note that the addition of TGF-β primed CD4+ cells to responder T cells markedly decreased cell division by CD8+ cells.

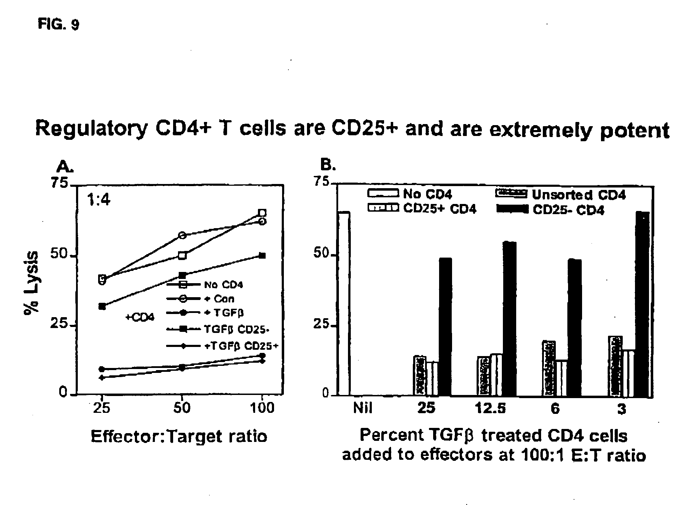

Figure 9 depicts that regulatory CD4+ cells express CD25+ (IL-2) receptors on their surface. Control and TGF-β induced CD4+ regulatory T cells were prepared as described above. After conditioning with allo-stimulator cells and TGF-β, the CD4+ cells were divided into CD25+ and CD25- subsets by cell sorting and added to fresh responder T cells and irradiated stimulator cells. The capacity of these responder cells to kill stimulator T lymphoblasts is shown in a standard 4 hour chromium release assay.
In Figure 9A, the open boxes show CTL activity without additional CD4+ cells. Control or TGF-β induced regulatory T cells were added in a 1:4 ratio with responder cells. The open circles show that the control CD4+ cells did not alter CTL activity. The solid circles show that TGF-β induced CD4+ cells almost completely suppressed CTL activity. The solid diamonds show that the suppressive activity was contained exclusively in the CD25+ subset. The CD25- subset (solid squares) did not have suppressive activity.
Figure 9B shows the effect of decreasing the numbers of CD4+ regulatory cells added to the MLR. Decreasing the number to only 3% had a minimal effect in decreasing the suppressive effects.

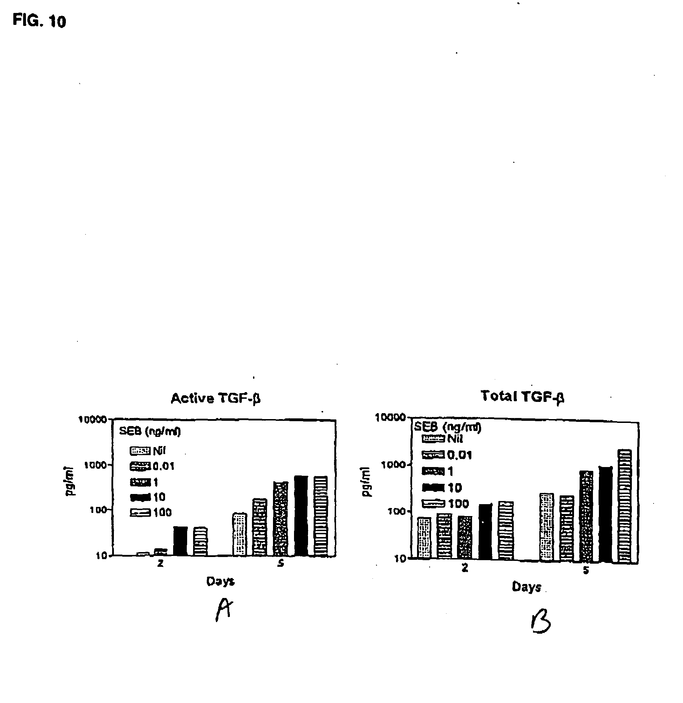

Figure 10 depicts that repeated stimulation of T cells with a low dose of staphylococcal enterotoxin B (SEB) induces T cells to produce immunosuppressive levels of TGF-β. CD4+ T cells were stimulated with SEB (0.01 ng/ml) and irradiated B cells as superantigen presenting cells with our without TGF-β at the times indicated by the arrows. Active TGF-β was measured 2 or 5 days later.

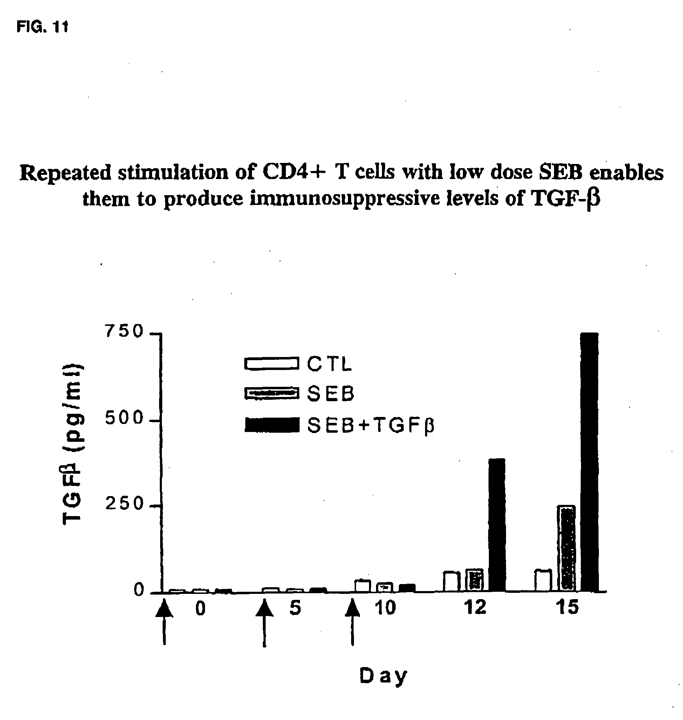

Figure 11 depicts that repeated stimulation of CD4+ T cells with a low dose of SEB enables these cell is to produce immunosuppressive levels of TGF-β. CD4+ T cells were stimulated with SEB (0.01 ng/ml) and irradiated B cells as superantigen presenting cells with or without TGF-β at the times indicated by the arrows. Active TGF-β was measured 2 or 5 days later.

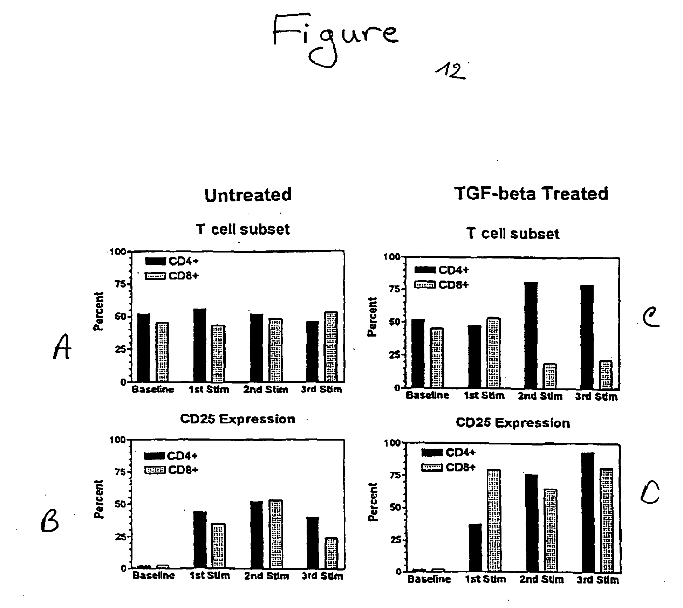

Figure 12 shows the effects of SEB on naive (CD45RA+ CD45RO-) CD4+ and CD8+ T cells. The cells were stimulated with SEB every 5th day for a total of three stimulations. The percentages of each T cell subset and the cells expressing the CD25 IL-2 receptor activation marker were determined after each stimulation. Panels A and C show that if TGF-β 1ng/ml was included in the initial stimulation, CD4+ T cells became the predominant subset in the cultures after repeated stimulation. Panels B and D show that CD25 expression by SEB stimulated cells decreases by the third stimulation in control cultures. However, CD25 expression remains high if the T cells have been primed with TGF-β.

### DETAILED DESCRIPTION

The compositions employed in the medical useS disclosed herein enhance cell mediated immune responses that are frequently defective in patients with cell mediated autoimmune disorders; that is, patients with cell mediated autoimmune disorders can be treated to ameliorate their defective cell-mediated symptoms.

Alternatively, the compositions are used to treat cell-mediated autoimmune disease. In this embodiment, the compositions induce immune cells to generate suppressor T cells. These suppressor T cells prevent other T cells from becoming cytotoxic and attacking the cells and tissue of an affected individual. Thus, the composition decrease cytotoxicity and thereby ameliorate the symptoms of cell-mediated autoimmune disorders.

This strategy is unlike almost all other treatment modalities currently in use which are either anti-inflammatory or immunosuppressive. Commonly used corticosteroids suppress cytokine production and block the terminal events which cause tissue injury, but generally do not alter the underlying autoimmune response. Cytotoxic drugs or experimental genetically engineered biologicals such as monoclonal antibodies may also deplete specific lymphocyte populations or interfere with their function. These drugs are generally only moderately successful and have severe adverse side effects. Certain cytokines have been given systemically to patients, but these agents also have broad actions with associated serious adverse side effects.

By contrast, the strategy of the present invention is to produce remission by restoring normal regulatory cell function and, thus, "resetting" the immune system. Another significant potential advantage of this strategy is a low probability of serious adverse side effects. Since only trace amounts of regulatory compositions such as cytokines will be returned to the patient, there should be minimal toxicity.

Circulating B lymphocytes spontaneously secreting IgG are increased in patients with active SLE (Blaese, R.M., et al. (1980), Am J. Med 69:345-350; Klinman, D.M. et al. (1991) Arthritis Rheum 34: 1404-1410). Sustained production of polyclonal IgG and autoantibodies in vitro requires T cell help (Shivakumar, S. et al. (1989), J Immunol 143:103-112). Previous studies of T cell regulation of spontaneous IgG production shows that while CD8+ T cells inhibit antibody production in healthy individuals, in SLE these cells support B cell function instead (Linker-Israeli, M. et al. (1990), Arthritis Rheum 33:1216-1225). In other autoimmune diseases such as rheumatoid arthritis and mutliple sclerosis, T cells rather than antibody are responsible for tissue injury and the resulting inflammation (Panayi GS, et al. Arthritis Rheum (1992) 35:725-773), Allegretta M et al. Science (1990) 247:718-722.

Accordingly, in a preferred embodiment, the present invention relates to treating cell-mediated autoimmune diseases that comprise removing peripheral blood mononuclear cells (PBMCs) from the patient with the autoimmune disease and treating certain of these cells with an regulatory composition.

Without being bound by theory, it appears there are several ways the methods of the invention may work. First of all, the treatment of the cells by an regulatory composition leads to the direct suppression of antibody production in the treated cells, which can lead to amelioration of antibody-mediated autoimmune symptoms. Alternatively or additionally, the treatment of the cells induces regulatory cells to down regulate antibody production in other cells. Antibody in this context includes all forms of antibody, including IgA, IgM. IgG, IgE, *etc.* The net result is a decrease in the amount of antibody in the system.

Additionally, the treatment of the cells enhances cell-mediated immune responses in patients with antibody-mediated autoimmune symptoms. Without being bound by theory, it appears that the treatment of the cells restores the balance between IL-10 and TNF-α leading to an enhanced production of Th1 cytokines and normalization of cell mediated immunity.

Furthermore, stimulation of immune cells with regulatory compositions including TGF-β can suppress cell-mediated immune responses. Without being bound by theory, it appears that CD4+ T cells can be stimulated to produce immunosuppressive levels of active TGF-β, that then suppresses cell-mediated immune responses. Alternatively, CD4+ T cells can be stimulated to suppress the activation and/or effector functions of other T cells by a contact-dependent mechanism of action. These effects require CD4+ cells to be activated in the presence of TGF-β.

Thus, the present invention inhibits aberrant immune responses. In patients with antibody-mediated autoimmune disorders, the present invention restores the capacity of peripheral blood T cells to down regulate antibody production and restores cell mediated immune responses by treating them with an regulatory composition ex vivo. In patients with cell-mediated disorders, the present invention generates regulatory T cells which suppress cytotoxic T cell activity in other T cells.

By "immune response" herein is meant host responses to foreign or self antigens. By "aberrant immune responses" herein is meant the failure of the immune system to distinguish self from non-self or the failure to respond to foreign antigens. In other words, aberrant immune responses are inappropriately regulated immune responses that lead to patient symptoms. By "inappropriately regulated" herein is meant inappropriately induced, inappropriately suppressed and/or non-responsiveness. Aberrant immune responses include, but are not limited to, tissue injury and inflammation caused by the production of antibodies to an organism's own tissue, impaired production of IL-2, TNF-α and IFN-γ and tissue damage caused by cytotoxic or non-cytotoxic mechanisms of action.

By "defects in cell mediated immune response" herein is meant impaired host defense against infection. Impaired host defense against infection includes, but is not limited to, impaired delayed hypersensitivity, impaired T cell cytotoxicity and impaired production of TGF-β. Other defects, include, but are not limited to, increased production of IL-10 and decreased production of IL-2, TNF-α and IFN-γ. Using the methods of the present invention, purified T cells are stimulated to increase production of IL-2, TNF-α and IFN-γ and decrease production of IL-10. T cells which can be stimulated using the current methods include, but are not limited to, CD4+ and CD8+.

The present invention provides methods of treating cell-mediated autoimmune disorders in a patient. By "cell-mediated autoimmune diseases" herein is meant a disease in which the cells of an individual are activated or stimulated to become cytotoxic and attack their own cells or tissues. Alternatively, the autoimmune cells of the individual may stimulate other cells to cause tissue damage by cytotoxic or non-cytotoxic mechanisms of action. Cell-mediated autoimmune diseases include, but are not limited to, Hashimoto's disease, polymyositis, disease inflammatory bowel disease, multiple sclerosis, diabetes mellitus, rheumatoid arthritis, and scleroderma.

By "treating" an autoimmune disorder herein is meant that at least one symptom of the autoimmune disorder is ameliorated by the methods outlined herein. This may be evaluated in a number of ways, including both objective and subjective factors on the part of the patient. For example, immunological manifestations of disease can be evaluated; for example, the level of spontaneous antibody and autoantibody production, particularly IgG production in the case of SLE, is reduced. Total antibody levels may be measured, or autoantibodies, including, but not limited to, anti-double-stranded DNA (ds DNA) antibodies, anti-nucleoprotein antibodies, anti-Sm, anti-Rho, and anti-La. Cytotoxic activity can be evaluated as outlined herein. Physical symptoms may be altered, such as the disappearance or reduction in a rash in SLE. Renal function tests may be performed to determine alterations; laboratory evidence of tissue damage relating to inflammation may be evaluated. Decreased levels of circulating immune complexes and levels of serum complement are further evidence of improvement. In the case of SLE, a lessening of anemia may be seen. The ability to decrease a patient's otherwise required drugs such as immunosuppressives can also be an indication of successful treatment. Other evaluations of successful treatment will be apparent to those of skill in the art of the particular autoimmune disease.

By "patient" herein is meant a mammalian subject to be treated, with human patients being preferred. In some cases, the methods of the invention find use in experimental animals, in veterinary application, and in the development of animal models for disease, including, but not limited to, rodents including mice, rats, and hamsters; and primates.

The methods provide for the removal of blood cells from a patient. In general, peripheral blood mononuclear cells (PBMCs) are taken from a patient using standard techniques. By "peripheral blood mononuclear cells" or "PBMCs" herein is meant lymphocytes (including T-cells, B-cells, NK cells, etc.) and monocytes. As outlined more fully below, it appears that in one embodiment, the main effect of the regulatory composition is to enable CD8+ orCD4+T lymphocytes to suppress harmful autoimmune responses. Accordingly, the PBMC population should comprise CD8+ T cells. Preferably, only PBMCs are taken, either leaving or returning substantially all of the red blood cells and polymorphonuclear leukocytes to the patient This is done as is known in the art, for example using leukophoresis techniques. In general, a 5 to 7 liter leukophoresis step is done, which essentially removes PBMCs from a patient, returning the remaining blood components. Collection of the cell sample is preferably done in the presence of an anticoagulant such as heparin, as is known in the art.

In some embodiments, a leukophoresis step is not required.

In general, the sample comprising the PBMCs can be pretreated in a wide variety of ways. Generally, once collected, the cells can be additionally concentrated, if this was not done simultaneously with collection or to further purify and/or concentrate the cells. The cells may be washed, counted, and resuspended in buffer.

The PBMCs are generally concentrated for treatment, using standard techniques in the art. In a preferred embodiment, the leukophoresis collection step results a concentrated sample of PBMCs, in a sterile leukopak, that may contain reagents and/or doses of the regulatory composition, as is more fully outlined below. Generally, an additional concentration/purification step is done, such as Ficoll-Hypaque density gradient centrifugation as is known in the art.

In a preferred embodiment, the PBMCs are then washed to remove serum proteins and soluble blood components, such as autoantibodies, inhibitors, *etc.,* using techniques well known in the art. Generally, this involves addition of physiological media or buffer, followed by centrifugation. This may be repeated as necessary. They can be resuspended in physiological media, preferably AIM-V serum free medium (Life Technologies) (since serum contains significant amounts of inhibitors) although buffers such as Hanks balanced salt solution (HBBS) or physiological buffered saline (PBS) can also be used.

Generally, the cells are then counted; in general from 1 X 10⁹ to 2 X 10⁹ white blood cells are collected from a 5-7 liter leukophoresis step. These cells are brought up roughly 200 mls of buffer or media.

In a preferred embodiment, the PBMCs may be enriched for one or more cell types. For example, the PBMCs may be enriched for CD8+ T cells or CD4+ T cells. This is done as is known in the art. as described in Gray et al. (1998), J. Immunol. 160:2248, Generally, this is done using commercially available immunoabsorbent columns, or using research procedures (the PBMCs are added to a nylon wool column and the eluted, nonadherent cells are treated with antibodies to CD4, CD16, CD11b and CD74, followed by treatment with immunomagnetic beads, leaving a population enriched for CD8+ T cells).

In a preferred embodiment, the PBMCs are separated in a automated, closed system such as the Nexell lsolex 300i Magnetic Cell Selection System. Generally, this is done to maintain sterility and to insure standardization of the methodology used for cell separation, activation and development of suppressor cell function.

Once the cells have undergone any necessary pretreatment, the cells are treated with a regulatory composition. By "treated" herein is meant that the cells are incubated with the regulatory composition for a time period sufficient to develop the capacity to inhibit immune responses, including antibody and autoantibody production, particularly when transferred back to the patient. The incubation will generally be under physiological temperature. As noted above, this may happen as a result of direct suppression of Antibody production by the treated cells, or by inducing regulatory cells to down regulate the production of antibody in the patient's lymphoid organs.

By "regulatory composition" or "antibody production inhibitor composition" or "humoral inhibitor composition" or "non-specific immune cell inhibitor" or specific T cell inhibitor" or "inhibitory composition" or "suppressive composition" herein is meant a composition that can cause suppression of immune responses, including inhibition of T cell activation, inhibition of spontaneous antibody and autoantibody production, or cytotoxicity, or both. Generally, these compositions are cytokines. Suitable regulatory compositions include, but are not limited to, T cell activators such as anti-CD2, including anti-CD2 antibodies and the CD2 ligand, LFA-3, and mixtures or combinations of T cell activators such as Concanavalin A (Con A), staphylococcus enterotoxin B (SEB), anti-CD3, anti-CD28 and cytokines such as IL-2. IL-4, TGF-β and TNF-α. A preferred regulatory composition for antibody suppression is a mixture containing a T cell activator, IL-2 and TGF-β. The preferred regulatory composition for suppression of cytotoxicity is TGF-β.

The concentration of the regulatory composition will vary on the identity of the composition. In a preferred embodiment, TFG-β is a component the regulatory composition. By "transforming growth factor -β" or "TGF-β" herein is meant any one of the family of the TGF-βs, including the three isoforms TGF-β1, TGF-β2, and TGF-β3: see Massague, J. (1980), J. Ann. Rev. Cell Biol 6:597. Lymphocytes and monocytes produce the β1 isoform of this cytokine (Kehrl, J.H. et al. (1991), Int J Cell Cloning 9: 438-450). The TFG-β can be any form of TFG-β that is active on the mammalian cells being treated.

In humans, recombinant TFG-β is currently preferred. A preferred human TGF-β can be purchased from Genzyme Pharmaceuticals, Farmington, MA. In general, the concentration of TGF-β used ranges from about 2 picograms/ml of cell suspension to about 5 nanograms, with from about 10 pg to about 4 ng being preferred, and from about 100 pg to about 2 ng being especially preferred, and 1 ng/ml being ideal.

In a preferred embodiment, IL-2 is used in the regulatory composition. The IL-2 can be any form of IL-2 that is active on the mammalian cells being treated. In humans, recombinant IL-2 is currently preferred. Recombinant human IL-2 can be purchased from Cetus, Emeryville, CA. In general, the concentration of IL-2 used ranges from about 1 Unit/ml of cell suspension to about 100 U/ml, with from about 5 U/ml to about 25 U/ml being preferred, and with 10 U/ml being especially preferred. In a preferred embodiment, IL-2 is not used alone.

In a preferred embodiment, CD2 activators, such as a combination of mitogenic anti CD2 antibodies, which may include the CD2 ligand LFA-3, are used as the regulatory composition. CD2 is a cell surface glycoprotein expressed by T lymphocytes. By "CD2 activator" herein is meant compound that will initiate the CD2 signaling pathway. A preferred CD2 activator comprises anti CD2 antibodies (OKT11, American Type Culture Collection, Rockville MD and GT2, Huets, et al., (1986) J. Immunol. 137:1420). In general, the concentration of CD2 activator used will be sufficient to induce the production of TGF-β. The concentration of anti CD2 antibodies used ranges from about 1 ng/ml to about 10 µg/ml, with from about 10 ng/ml to about 100 ng/ml being especially preferred.

In some embodiments it is desirable to use a mitogen to activate the cells; that is, many resting phase cells do not contain large amounts of cytokine receptors. The use of a mitogen such as Concanavalin A or staphylococcus enterotoxin B (SEB) can allow the stimulation of the cells to produce cytokine receptors, which in turn makes the methods of the invention more effective. When a mitogen is used, it is generally used as is known in the art, at concentrations ranging from 1 µg/ml to about 10 µg/ml is used. In addition, it may be desirable to wash the cells with components to remove the mitogen, such as α-methyl mannoside, as is known in the art.

In a preferred embodiment, T cells are strongly stimulated with mitogens, such as anti-CD2, anti-CD3, anti-CD28 or combinations of monoclonal antibodies, or a specific autoantigen, if known, and anti-CD28 or IL-2 as a co-stimulator. ConA is also used to stimulate T cells. The presence of TGF-β in the suppressive composition induces T cells to develop potent suppressive activity. Repeated stimulation of the T cells with our without TGF-β in secondary cultures may be necessary to develop maximal suppressive activity.

In a preferred embodiment, the invention provides methods comprising conditioning T cells, including, but not limited to CD8+ T or CD4+ T cells. and other minor T cell subsets such as CD8CD4, NKT etc., with TGF-β. These T cells prevent other T cells from becoming cytotoxic effector cells.

In a preferred embodiment, the invention provides methods comprising conditioning CD4+ or CD8+ T cells with TGF-β to produce immunosuppresive levels of TGF-β

In a preferred embodiment, the invention provides methods comprising conditioning CD4+ or CD8+ T cells with TGF-β to produce T cells that suppress by a contact-dependent mechanism.

In a preferred embodiment, the invention provides methods comprising treating naive CD4+ T cells with a stimulant such that said CD4+ cells produce immunosuppressive levels of active TGF-β. By "stimulant" is generally meant a generalized stimulant that triggers all T cells, such as anti-CD2 or anti-CD3.

In a preferred embodiment, the invention provides methods comprising stimulating naive CD4+ T cells in the presence of TGF-β to expand the CD4+ cell population.

In a preferred embodiment, the invention provides methods which decrease production of IL-10 and correspondingly increase TNF-α production.

The regulatory composition is incubated with the cells for a period of time sufficient to cause an effect. In a preferred embodiment, treatment of the cells with the regulatory composition is followed by immediate transplantation back into the patient. Accordingly, in a preferred embodiment, the cells are incubated with the regulatory composition for 12 hours to about 7 days. The time will vary with the suppressive activity desired. For suppression of antibody production 48 hours is especially preferred and 5 days is especially preferred for suppression of cytotoxicity.

In one embodiment, the cells are treated for a period of time, washed to remove the regulatory composition, and may be reincubated to expand the cells. Before introduction into the patient, the cells are preferably washed as outlined herein to remove the regulatory composition. Further incubations for testing or evaluation may also be done, ranging in time from a few hours to several days. If evaluation of antibody production prior to introduction to a patient is desirable, the cells will be incubated for several days to allow antibody production (or lack thereof) to occur.

Once the cells have been treated, they may be evaluated or tested prior to autotransplantation back into the patient. For example, a sample may be removed to do: sterility testing: gram staining, microbiological studies; LAL studies; mycoplasma studies; flow cytometry to identify cell types; functional studies, *etc.* Similarly, these and other lymphocyte studies may be done both before and after treatment.

In a preferred embodiment, the quantity or quality, *i.e.* type, of antibody production, may be evaluated. Thus, for example, total levels of antibody may be evaluated, or levels of specific types of antibodies, for example, IgA, IgG, IgM, anti-DNA autoantibodies, anti-nucleoprotein (NP) antibodies, *etc.* may be evaluated. Regulatory T cells may also be assessed for their ability to suppress T cell activation or to prevent T cell cytotoxicity against specific target cells in vitro.

In a preferred embodiment, the levels of antibody, particularly IgG, are tested using well known techniques, including ELISA assays, as described in Abo et al. (1987), Clin. Exp. Immunol. 67:544 and Linker-Israeli et al. (1990), Arthritis Rheum 33:1216. These techniques may also be used to detect the levels of specific antibodies, such as autoantibodies.

In a preferred embodiment, the treatment results in a significant decrease in the amount of IgG and autoantibodies produced, with a decrease of at least 10% being preferred, at least 25% being especially preferred, and at least 50% being particularly preferred. In many embodiments, decreases of 75% or greater are seen.

In a preferred embodiment, prior to transplantation, the amount of total or active TGF-β can also be tested. As noted herein, TGF-β is made as a latent precursor that is activated post-translationally.

After the treatment, the cells are transplanted or reintroduced back into the patient. This is generally done as is known in the art, and usually comprises injecting or introducing the treated cells back into the patient, via intravenous administration, as will be appreciated by those in the art. For example, the cells may be placed in a 50 ml Fenwall infusion bag by injection using sterile syringes or other sterile transfer mechanisms. The cells can then be immediately infused via IV administration over a period of time, such as 15 minutes, into a free flow IV line into the patient. In some embodiments, additional reagents such as buffers or salts may be added as well.

After reintroducing the cells into the patient, the effect of the treatment may be evaluated, if desired, as is generally outlined above. Thus, evaluating immunological manifestations of the disease may be done; for example the titers of total antibody or of specific immunoglobulins, renal function tests, (issue damage evaluation, *etc.* may be done. Tests of T cells function such as T cell numbers, phenotype, activation state and ability to respond to antigens and/or mitogens also may be done.

The treatment may be repeated as needed or required. For example, the treatment may be done once a week for a period of weeks, or multiple times a week for a period of time, for example 3-5 times over a two week period. Generally, the amelioration of the autoimmune disease symptoms persists for some period of time, preferably at least months. Over time, the patient may experience a relapse of symptoms, at which point the treatments may be repeated.

In a preferred embodiment, the invention further provides kits for the practice of the methods of the invention, *i.e.,* the incubation of the cells with the regulatory compositions. The kit may have a number of components. The kit comprises a cell treatment container that is adapted to receive cells from a patient with an antibody-mediated or cell-mediated autoimmune disorder. The container should be sterile. In some embodiments, the cell treatment container is used for collection of the cells, for example it is adaptable to be hooked up to a leukophoresis machine using an inlet port. In other embodiments, a separate cell collection container may be used.

In a preferred embodiment, the kit comprises a cell treatment container that is adapted to receive cells from a patient with a cell mediated disorder. The kit may also be adapted for use in a automated closed system to purify specific T cell subsets and expand them for transfer back to the patient.

The form and composition of the cell treatment container may vary, as will be appreciated by those in the art. Generally the container may be in a number of different forms, including a flexible bag, similar to an IV bag, or a rigid container similar to a cell culture vessel. It may be configured to allow stirring. Generally, the composition of the container will be any suitable, biologically inert material, such as glass or plastic, including polypropylene, polyethylene, *etc.* The cell treatment container may have one or more inlet or outlet ports, for the introduction or removal of cells, reagents, regulatory compositions, *etc.* For example, the container may comprise a sampling port for the removal of a fraction of the cells for analysis prior to reintroduction into the patient. Similarly, the container may comprise an exit port to allow introduction of the cells into the patient; for example, the container may comprise an adapter for attachment to an IV setup.

The kit further comprises at least one dose of an regulatory composition. "Dose" in this context means an amount of the regulatory composition such as cytokines, that is sufficient to cause an effect. In some cases, multiple doses may be included. In one embodiment, the dose may be added to the cell treatment container using a port; alternatively, in a preferred embodiment, the dose is already present in the cell treatment container. In a preferred embodiment, the dose is in a lyophilized form for stability, that can be reconstituted using the cell media, or other reagents.

In some embodiments, the kit may additionally comprise at least one reagent, including buffers, salts, media, proteins, drugs, *etc.* For example, mitogens, monoclonal antibodies and treated magnetic beads for cell separation can be included.

In some embodiments, the kit may additional comprise written instructions for using the kits.

The following examples serve to more fully describe the manner of using the above-described invention, as well as to set forth the best modes contemplated for carrying out various aspects of the invention. It is understood that these examples in no way serve to limit the true scope of this invention, but rather are presented for illustrative purposes.

### Reagents

Recombinant TGF-β and monoclonal anti-TGF-β (1D11.16) antibody, a murine IgG1, were kindly provided by Dr. Bruce Pratt (Genzyme Pharmaceuticals. Farmington, MA). Recombinant IL-10 and monoclonal anti-lL-10 (JES3-19F1) antibody, and control rat IgG2a, were kindly provided by Dr. Satwant Narula (Schering Plough Pharmaceuticals, Kenilworth, NJ). Control murine IgG1 myeloma protein was purchased from Calbiochem, San Diego, CA. Recombinant human IL-2 was purchased from Chiron, Emmeryville, CA. Anti-CD2 secreting hybridomas antibodies used OKT11 were obtained from the American Type Culture Collection (ATCC), Rockville, MD and GT2 was generously provided by A. Bernard. Nice, France). Other antibodies included: anti-CD4 (OKT4, ATCC), anti-CD8 (OKTB. ATCC: CD8, Dako. Carpenteria, CA), anti-CD11b (OKM1, ATCC), anti-CD16 (3G8), kindly provided by J. Unkeless, New York, NY); anti-CD20 (Leu 16, Becton Dickinson, San Jose, CA) and anti-CD74 (L243, ATCC).

### Isolation of blood mononuclear cells

Peripheral blood mononuclear cells (PBMC) were prepared from heparinized venous blood by Ficoll. Hypaque^{™} (Pharmacia, Piscataway, NJ) density gradient centrifugation. The mononuclear cells were washed in PBS with 5mM EDTA (Life Technologies, Grand Island, NY) to remove platelets, which are a rich source of TGF-β.

### Cell culture procedures

Procedures for cell cultures have been described previously (Wahl, S.M. (1994), J Exp Med 180:1587-1590; Gray. J.D. et al. (1998), J Immunol 160:2248-2254). In brief, 2x10⁵ of PBMC were cultured in serum-free AIM-V culture medium (Life Technologies) in the wells of 96-well flat bottom microtiter plate with or without the indicated cytokines. After three days of culture, the PBMC were washed three times then fresh serum-free medium was added. After a further 7 days at 37°C, supernatants were harvested and assayed for total IgG and autoantibodies reactive with calf thymus nucleoprotein (NP) by a solid phase enzyme-linked immunoadsorbant assay (ELISA), as described previously (Linker-Israeli, M. et al. (1990), Arthritis Rheum 33:1216-1225). The optical density (OD) readings were transformed into units/ml (U/ml) from a standard curve using positive and negative standards. Supernatants from PBMC culture of SLE patients (with high titers of anti-NP antibodies) and normal individuals were used as controls.

### Statistical analysis

The data were analyzed using Graph Pad, Prism software (San Diego, CA). We used analysis of variance (ANOVA) after log transformation of the data and the non-parametric Mann-Whitney test.

### Example 1

### Treating Cells to Normalize Cell-mediated Immunity

Contributing to autoantibody production in SLE is an imbalance between IL-10 and TNF-α production. Levels of IL-10 are excessive and levels of TNF-α are decreased (Llorente et al. 1995. J Exp. Med. 181:839-44) (Houssiau. F.A. et al., 1995. Lupus 4:393-5. (Ishida, H. et al. 1994. J Exp. Med. 179:305-10) (Jacob, C.O. and McDevitt, H.O., 1988. Nature 331:356-358). We have evidence that this imbalance is corrected by strongly activating T cells in the presence of TGF-β and have recently elucidated the mechanism of action of this effect.

Purified T cells were prepared as outlined above, and incubated with ConA and IL-2 with or without TGF-β. Figure 1 shows that T cell stimulation in the absence of TGF-β, resulted in increased production of IL-10. However, when TGF-β was added to stimulated T cells, IL-10 production was blocked and production of TNF-α was increased. In addition, TNFR2 expression was increased significantly. Without being bound by theory, It is believed that accelerated TNF-α signaling via TNFR₂ induced by TGF-β results in regulatory T cells that inhibit antibody production. Our results support this suggestion.

We have determined that upregulation of TNF-α by TGF-β is essential for the induction of regulatory T cells. Figure 2 shows two experiments where the addition of TGF-β to activated CD8+ T cells resulted in marked suppression of IgG production. This suppressive activity depended upon TNF-α as an essential intermediate, in each of these experiments, a neutralizing anti-TNF-α antibody completely abolished the suppressive effects of the CD8+ regulatory T cells (CDBreg).

Patients with SLE have a marked defect in cell-mediated immunity with impaired production of IL-2. TNF-α and IFN-γ. (Horwitz, D.A. et al. (1997), Dubois' Lupus Erythematosus, 5th Ed. (1997), pp. 83-96, D.J. Wallace et al. eds., Williams and Wilkins, Baltimore). Without being bound by theory, it is believed that the defect in lymphocyte production of TGF-β is partially responsible for impaired production of IL-2, TNF-α and IFN-γ. We have found that stimulation of T cells in the presence of TGF-β significantly increased production of IL-2, TNF-α and IFN-γ when these cells were restimulated. Moreover, this result was dependent upon upregulation of TNF-α by TGF-β (see Figure 8).

We have evidence that TGF-β production is decreased in SLE and that this defect contributes to the imbalance between IL-10 and TNF-α. Without being bound by theory, it is believed that high levels of IL-10 in SLE sustain autoantibody production and are responsible for decreased production of TNF-α, IL-2, IFN-γ. Decreased production of these cytokines is responsible for defective cellular immunity in SLE. We have demonstrated that under specified conditions, TGF-β down-regulates IL-10 and enhances the production of TNF-α. Down-regulation of IL-10 and enhancement of TNF-α production by TGF-β plays a crucial role in the normalization of regulatory T cell activity in SLE, restoration of cell-mediated immunity and remission of disease.

### Example 2

### Generation of regulatory T cells that suppress cell-mediated autoimmunity

The previous examples used regulatory compositions to treat antibody-mediated autoimmune diseases. Similar compositions are used to induce CD4+ as well as CD6+ T cells to suppress cell-mediated autoimmune diseases. We show that CD8+ or CD4+ cells conditioned by TGF-β alone suppressed the generation of T cell cytotoxicity.

Instead of using mitogens to induce regulatory T cells, the allogeneic mixed lymphocyte reaction is used for this purpose. In this reaction. T cells from one individual recognize and respond to foreign histocompatibility antigens displayed by other individuals PBMCs. These responder T cells proliferate and develop the capacity to kill these target cells.

To develop suppressor T celts, various CD4+ and CD8+ T cell subsets from one individual (donor A) were cultured with irradiated T cell-depleted mononuclear cells from another individual (donor B). The cells were cultured for 5 days with or without TGF-β (1ng/ml) in the suspensions. After this time. TGF-β was removed and the cells added to fresh T cells from donor A and non-T cells from donor B. Figure 4 shows TGF-β induced both CD4+ and CD8+ T cell subsets to develop the capacity to inhibit cell mediated cytotoxicity. Figure 5 shows two additional experiments with CD4+ regulatory T cells induced by TGF-β.

Further studies revealed that regulatory CD4+ T cells generated in this manner have a unique mode of action. Unlike the CD8+ and CD4+ T cells generated previously which suppress by secreting inhibitory cytokines, these allo-specific regulatory CD4+ T cells have a contact dependent mechanism of action (Figure 6). Without being bound by theory, it is believed that these regulatory T cells suppress other T cells from being activated. Addition of these T cells to responder T cells and allo-stimulator cells inhibited proliferation (Figure 7) and decreased the ability of responder CD8+ killer precursor cells to become activated Figure 8).

We also learned that these regulatory CD4+ cells express IL-2 receptors (CD25) on their cell surface and were extremely potent (Figure 9). Decreasing the proportion of regulatory CD4+ cells to responder T cells from 1:4 (20%) to 1:32 (3%) only minimally decreased the inhibitory effects of these cells.

Because only a few of these cells are needed for potent down-regulatory effects, it is likely that a sufficient number can be transferred to patients to suppress autoimmunity or other desired immunosuppressive effects, such as inhibiting of graft rejection.

### Example 3

### Stimulating CD4+ T Cells to Produce Immunosuppressive Levels of TGF-β

CD4+ T cells that produce immunosuppressive levels of TGF-β have been named Th3 cells, but the mechanisms involved in their development are poorly understood. We have obtained evidence that strong stimulation of CD4+ cells with the superantigen, staphylococcus enterotoxin B (SEB), or repeated stimulation of CD4+ cells stimulated with a lower concentration of SEB induced these cells to produce immunosuppressive levels of active TGF-β.

Figure 10 shows increased production of both active and total TGF-β produced by CD4+ T cells stimulated with increasing concentrations of SEB. Figure 11 shows the effect of repeated stimulation of CD4+ T cells with low doses of SEB. By the third time these T cells were stimulated with SEB, they produced significant amounts of the active form of TGF-β.

Figure 12 shows the effects of SEB on naive (CD45RA+ CD45RO-) CD4+ and CD8+ T cells. The cells were stimulated with SEB every 5th day for a total of three stimulations. The percentages of each T cell subset and the cells expressing the CD25 IL-2 receptor activation marker were determined after each stimulation. Panels A and C show that by including TGF-β 1ng/ml in the initial stimulation. CD4+ T cells became the predominant subset in the cultures after repeated stimulation. Panels B and D show that CD25 expression by SEB stimulated cells decreased by the third stimulation in control cultures. However, CD25 expression remained very high if the T cells were primed with TGF-β. Thus, TGF-β appears to have preferential effects on CD4+ cells if these T cells are repeatedly stimulated and almost all of these cells were CD25+ after culture for 20 days.

In summary, following T cell stimulation, the predominant regulatory effects of TGF-β are directed to CD8+ cells. Upon repeated stimulation, this cytokine now induces CD4+ cells to become regulatory cells and these cells are more potent than CD8+ cells in their suppressive activities.

## Claims

1. Use of peripheral blood mononuclear cells (PBMC) which have been obtained from a patient with a cell-mediated autoimmune disorder and treated with a regulatory composition comprising TGF-β, for the preparation of a medicament for treating said cell-mediated autoimmune disorder in said patient.

2. The use of claim 1 wherein said medicament decreases the cytotoxcity associated with said cell-mediated disorder.

3. The use of claim 1 wherein said cell-mediated autoimmune disorder is selected from the group consisting of Hashimoto's disease, polymyositis disease, inflammatory bowel disease, multiple sclerosis, diabetes mellitus, rheumatoid arthritis, and scleroderma.

4. The use of any one of claims 1 to 3 wherein said PBMC are enriched for CD8+ T cells.

5. The use of any one of claims 1 to 3 wherein said PBMC are enriched for CD4+ cells.

6. The use of any one of claims 1 to 3 wherein said PBMC are enriched for naive CD4+ cells.

7. The use of any one of claims 1 to 6 wherein said regulatory composition comprises IL-2.

8. The use of any one of claims 1 to 7 wherein said regulatory composition further comprises an anti-CD2 antibody.

9. The use of any one of claims 1 to 7 wherein said regulatory composition further comprises an anti-CD3 antibody.

10. The use of any one of claims 1 to 7 wherein said regulatory composition further comprises an anti CD28 antibody.

11. The use of any one of claims 1 to 10, wherein said treated cells prevent other T cells in said patient from becoming cytotoxic effector cells.

12. The use of any one of claims 1 to 10, wherein said treated cells are induced to produce immunosuppressive levels of active TGF-β.

## Patentansprüche

1. Verwendung von peripheren einkernigen Blutzellen (PBMC), die von einem Patienten mit einer zellvermittelten Autoimmunerkrankung erhalten wurden, der mit einer Regulierungszusammensetzung behandelt wurde, die TGF-β umfasst, zur Herstellung eines Medikaments zur Behandlung der zellvermittelten Autoimmunerkrankung bei dem Patienten.

2. Verwendung nach Anspruch 1, worin das Medikament die mit der zellvermittelten Erkrankung assoziierte Zytotoxizität verringert.

3. Verwendung nach Anspruch 1, worin es sich bei der zellvermittelten Autoimmunerkrankung um eine Erkrankung handelt, die aus der aus der Hashimoto-Erkrankung, der Polymyositis-Erkrankung, der Reizdarm-Erkrankung, Multipler Sklerose, Diabetes mellitus, Gelenkrheumatismus und Skleroderma bestehenden Gruppe ausgewählt ist.

4. Verwendung nach einem der Ansprüche 1 bis 3, worin die PBMC an CD8+-T-Zellen angereichert sind.

5. Verwendung nach einem der Ansprüche 1 bis 3, worin die PBMC an CD4+-Zellen angereichert sind.

6. Verwendung nach einem der Ansprüche 1 bis 3, worin die PBMC an naiven CD4+-Zellen angereichert sind.

7. Verwendung nach einem der Ansprüche 1 bis 6, worin die Regulierungszusammensetzung IL-2 umfasst.

8. Verwendung nach einem der Ansprüche 1 bis 7, worin die Regulierungszusammensetzung weiters einen Anti-CD2-Antikörper umfasst.

9. Verwendung nach einem der Ansprüche 1 bis 7, worin die Regulierungszusammensetzung weiters einen Anti-CD3-Antikörper umfasst.

10. Verwendung nach einem der Ansprüche 1 bis 7, worin die Regulierungszusammensetzung weiters einen Anti-CD28-Antikörper umfasst.

11. Verwendung nach einem der Ansprüche 1 bis 10, worin die behandelten Zellen andere T-Zellen in dem Patienten daran hindern, zu zytotoxischen Effektorzellen zu werden.

12. Verwendung nach einem der Ansprüche 1 bis 10, worin die behandelten Zellen induziert werden, um immunsuppressive Ausmaße von aktivem TGF-β zu produzieren.

## Revendications

1. Utilisation de cellules mononucléaires de sang périphérique (PBMC) qui ont été obtenues d'un patient souffrant d'une maladie auto-immune provoquée par les cellules, et traitées avec une composition régulatrice comprenant TGF-β pour la préparation d'un médicament pour le traitement de ladite maladie auto-immune provoquée par les cellules chez ledit patient.

2. Utilisation de la revendication 1, où ledit médicament diminue la cytotoxicité associée à ladite maladie provoquée par les cellules.

3. Utilisation de la revendication 1 où ladite maladie auto-immune provoquée par les cellules est sélectionnée dans le groupe consistant en maladie de Hashimoto, maladie de polymyosite, maladie inflammatoire des intestins, sclérose en plaques, diabète sucré, arthrite rhumatoïde et sclérodermie.

4. Utilisation de l'une quelconque des revendications 1 à 3 où lesdites PBMC sont enrichies en cellules T CD8+.

5. Utilisation de l'une quelconque des revendications 1 à 3 où lesdites PBMC sont enrichies en cellules CD4+.

6. Utilisation de l'une quelconque des revendications 1 à 3 où lesdites PBMC sont enrichies en cellules CD4+ naïves.

7. Utilisation de l'une quelconque des revendications 1 à 6 où ladite composition régulatrice comprend IL-2.

8. Utilisation de l'une quelconque des revendications 1 à 7 où ladite composition régulatrice comprend de plus un anticorps anti-CD2.

9. Utilisation de l'une quelconque des revendications 1 à 7 où ladite composition régulatrice comprend de plus un anticorps anti-CD3.

10. Utilisation de l'une quelconque des revendications 1 à 7 où ladite composition régulatrice comprend de plus un anticorps anti-CD28.

11. Utilisation de l'une quelconque des revendications 1 à 10 où lesdites cellules traitées empêchent d'autres cellules T dudit patient de devenir des cellules cytotoxiques effectrices.

12. Utilisation de l'une quelconque des revendications 1 à 10 où lesdites cellules traitées sont induites à produire des niveaux immunosuppresseurs de TGF-β actif.
